(19) Europäisches Patentamt
European Patent Office
Office européen des brevets

(11) EP 2 378 978 B1

(12) **EUROPEAN PATENT SPECIFICATION**

(45) Date of publication and mention
of the grant of the patent:
**12.11.2014 Bulletin 2014/46**

(21) Application number: **09833984.9**

(22) Date of filing: **22.12.2009**

(51) Int Cl.:
*A61B 8/13* (2006.01)     *G06T 7/00* (2006.01)
*A61B 8/08* (2006.01)

(86) International application number:
**PCT/CA2009/001881**

(87) International publication number:
**WO 2010/071999 (01.07.2010 Gazette 2010/26)**

(54) **Method and system for automated generation of surface models in medical images**

Verfahren und System zur automatisierten generierung von oberflächenmodellen in medizinischen bildern

Méthode et système de génération automatique de modèles de surface dans des images médicales

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **22.12.2008  US 139723 P**

(43) Date of publication of application:
**26.10.2011  Bulletin 2011/43**

(60) Divisional application:
**13193330.1 / 2 712 554**

(73) Proprietor: **Salient Imaging, Inc.**
**Milford, NH 03055 (US)**

(72) Inventors:
• **RICO, Dan**
**Thornhill**
**Ontario L4J 7C5 (CA)**
• **CHUNG, Desmond Ryan**
**Thornhill**
**Ontario L4J 7R7 (CA)**

(74) Representative: **Holmes, Jacqueline Susan et al**
**Dummett Copp LLP**
**25 The Square**
**Martlesham Heath**
**Ipswich, Suffolk IP5 3SL (GB)**

(56) References cited:
EP-A1- 1 074 939     EP-A2- 1 136 914
WO-A2-99/04680     WO-A2-2006/057740
US-A- 5 830 141     US-B1- 6 173 034

**Description**

**Field of Invention**

The invention relates generally to the field of computerized processing of medical images. In particular, the invention relates to identification of tissue layers in medical images, automated detection of lesions in medical images and normalization of pixel intensities of medical images.

**Background of Invention**

Cancer is recognized as a leading cause of death in many countries. It is generally believed that early detection and diagnosis of cancer and therefore early treatment of cancer help reducing mortality rate. Various imaging techniques for detection and diagnosis of cancer, such as breast cancer, ovarian cancer, and prostate cancer, have been developed. For example, current imaging techniques for detection and diagnosis of breast cancer include mammography, MRI and sonography, among other techniques.

Sonography is an ultrasound-based imaging technique and is generally used for imaging soft tissues of the body. Typically, a transducer is used to scan the body of a patient. An ultrasound (US) image of body tissues and organs is produced from ultrasound echoes received by the transducer. Feature descriptors of shape, contour, margin of imaged masses and echogenicity are generally used in diagnosis of medical ultrasound images. Sonography has been shown to be an effective imaging modality in classification of benign and malignant masses.

However, experiences of a radiologist often play an important role in correct diagnosis of ultrasound images. Sensitivity and negative predictive values attainable by highly experienced experts may not always be attainable by less experienced radiologists. Moreover, scanning techniques strongly influence quantification and qualification of distinguishing features for malignant and benign lesions. Such strong influence also contributes to inconsistent diagnosis of ultrasound images among radiologists with different levels of experience.

In addition, consistent analysis of ultrasound images is further complicated by the variation in absolute intensities. Absolute intensities of tissue types vary considerably between different ultrasound images, primarily due to operator dependent variables, such as gain factor, configured by hardware operators during image acquisition. The gain factor plays an important role in determining the mapping from tissue echogenicity to grey pixel intensity. The settings of gain factor configured by different operators may vary widely between scans and consequently make consistent analysis of ultrasound images more difficult.

Another operator-dependent setting, time gain control (TGC) setting, is also closely related to the overall gain factor for an ultrasound image. TGC adjusts the echogenicity to intensity mapping as a function of tissue depth. Tissue depth is typically represented by the pixel y-coordinate. Lack of consistent TGC setting, or consistent compensation for inconsistent TGC settings, poses another challenge to consistent and unified image analysis. WO 2006/057740 discloses a method and system for determining candidate lesions within an ultrasound image.

To overcome the effect of operator dependence and improve diagnostic performance of breast sonography, computer-aided diagnosis (CAD) systems have been developed. One function of CAD systems is to automatically detect and demarcate suspicious regions in ultrasound images by applying computer-based image processing algorithms to the images. This is a very challenging task due to the abundance of specular noise and structural artifacts in sonograms. Variable image acquisition conditions make a consistent image analysis even more challenging. Additional challenges include the tumor-like appearance of normal anatomical structures in ultrasound images: Cooper ligaments, glandular tissue and subcutaneous fat are among the normal breast anatomy structures that often share many of the same echogenic and morphological characteristics as true lesions.

It is an object of the present invention to mitigate or obviate at least one of the above mentioned challenges.

**Summary of Invention**

The present invention is outlined in the claims. The current description outlines identification of tissue layers in medical images, automated detection of lesions in medical images and normalization of pixel intensities of medical images.

The current description outlines a system and method for processing medical images. Input medical images are normalized first, utilizing pixel intensities of control point tissues, including subcutaneous fat. Clustered density map and malignance probability map are generated from a normalized image and further analyzed to identify regions of common internal characteristics, or blobs, that may represent lesions. These blobs are analyzed and classified to differentiate possible true lesions from other types of non-malignant masses often seen in medical images.

The current description outlines a method of identifying suspected lesions in an ultrasound medical image. The method includes the steps of: computing an estimated representative fat intensity value of subcutaneous fat pixels in the medical image, calculating normalized grey pixel values from pixel values of the medical image utilizing a mapping relationship between a normalized fat intensity value and the representative fat intensity value to obtain a normalized image, identifying pixels in the normalized image forming distinct areas, each of the distinct areas having consistent internal characteristics, extracting descriptive features from each of the distinct areas, analyzing the extracted descriptive features of the each distinct area and assigning to the each distinct area a likelihood value of the each distinct area being a lesion, and identifying all distinct areas having likelihood values satisfying a pre-determined criteria as candidate lesions.

The current description outlines a system for automatically identifying regions in a medical image that likely correspond to lesions. The system includes an intensity unit, the intensity unit being configured to compute estimated intensities of control point tissues in the medical image from pixel values in the medical image and a normalization unit, the normalization unit being configured to generate a mapping relationship between an input pixel and a normalized pixel and convert a grey pixel value to a normalized pixel value to obtain a normalized image according to the mapping relationship; a map generation unit, the map generation unit assigning a parameter value to each pixel in an input image to generate a parameter map; a blob detection unit, the blob detection unit being configured to detect and demarcate blobs in the parameter map; a feature extraction unit, the feature extraction unit being configured to detect and compute descriptive features of the detected blobs; and a blob analysis unit, the blob analysis unit computing from descriptive features of a blob an estimated likelihood value that the blob is malignant and assigning the likelihood value to the blob.

In another aspect, there is provided a method of estimating grey scale intensity of a tissue in a digital or digitized medical image. The method includes the steps of: applying a clustering operation to intensity values of pixels of the medical image to group the intensity values into distinct intensity clusters, identifying one of the distinct intensity clusters as an intensity cluster corresponding to the tissue, estimating a representative grey scale intensity value of the intensity cluster from grey scale intensities of pixels of the intensity cluster; and assigning the representative grey scale intensity to the tissue.

The current description outlines a method of processing an ultrasound breast image. The method includes the steps of: constructing a layered model of breast, each pair of neighboring layers of the model defining a boundary surface between the each pair of neighboring layers, calibrating the model on a plurality of sample ultrasound breast images, each of the plurality of sample ultrasound breast images being manually segmented to identify the boundary surfaces in the sample ultrasound breast images, the calibrated model comprising parameterized surface models, each parameterized surface model comprising a set of boundary surface look-up tables (LUTs) corresponding to a discrete value of a size parameter, receiving an estimated value of the size parameter of the ultrasound breast image, establishing a new surface model corresponding to the estimated value of the size parameter from the parameterized surface models, the new surface model comprising a set of computed boundary surface LUTs corresponding to the estimated value of the size parameter, and computing estimated locations of boundary surfaces from the set of computed boundary surface LUTs of the new surface model to identify pixels of a primary layer in the ultrasound breast image.

The current description outlines a method of identifying lesions in an ultrasound breast image. The method includes the steps of: computing estimated locations of surfaces separating primary layer tissues, said primary layer tissues including tissues in a mammary zone; identifying pixels in the mammary zone; constructing a pixel characteristic vector (PCV) for each pixel in the mammary zone, said PCV including at least characteristics of a neighborhood of each said pixel, for each of the pixels in the mammary zone, computing a malignancy probability value from the PCV of each pixel, assigning to each of the pixels the malignancy probability value and identifying a pixel as a possible lesion pixel if its assigned malignancy probability value is above a threshold value, and reporting contiguous regions of all possible lesion pixels as potential lesions.

## Brief Description of Drawings

For the purposes of description, but not of limitation, the foregoing and other aspects of the invention are explained in greater detail with reference to the accompanying drawings, in which:

Figure **1** is a flow chart that shows steps of a process of automatically segmenting a medical image and detecting possible lesions;

Figure **2** illustrates schematically functional components of a CAD system for processing and diagnosing medical images, and for implementing the process shown in Figure 1;

Figure **3** shows steps of another process of automatically segmenting a medical image and classifying the segmented masses into lesion candidates and non-significant areas;

Figure **4** includes Fig. **4a,** which shows an input image before the application of a de-noising algorithm and Fig. **4b,**

which shows a smoothed image;

Figure **5** shows steps of a process of automatically detecting a mean fat intensity of subcutaneous fat in a medical image, the mean fat intensity being used in a normalization step in processes illustrated in Figure **1** and Figure **3;**

Figure **6** illustrates the typical structure of a breast ultrasound image;

Figure **7** is a flow chart that shows steps of a method of estimating the locations of primary tissue layers in a breast image;

Figure **8** shows an example of variations of mammary zone depth (MZD) values in a three-dimensional view;

Figure **9a** shows an example of a model MZD surface in a three-dimensional view and Figure **9b** shows a two-dimensional profile of the example model MZD surface shown in Figure **9a;**

Figure **10** includes Fig. **10a**, which shows an input ultrasound image before the application of a normalization operation, and Fig. **10b,** which shows a normalized image;

Figure **11** illustrates the grey-level mapping of pixel intensity values for the respective control point tissues in an 8-bit image;

Figure **12** is a flow chart showing steps of a process of generating a malignance map;

Figure **13** is a flow chart showing steps of an alternative process of automatically segmenting a medical image and classifying the segmented masses into lesion candidates and non-significant areas.

## Detailed Description of Embodiments

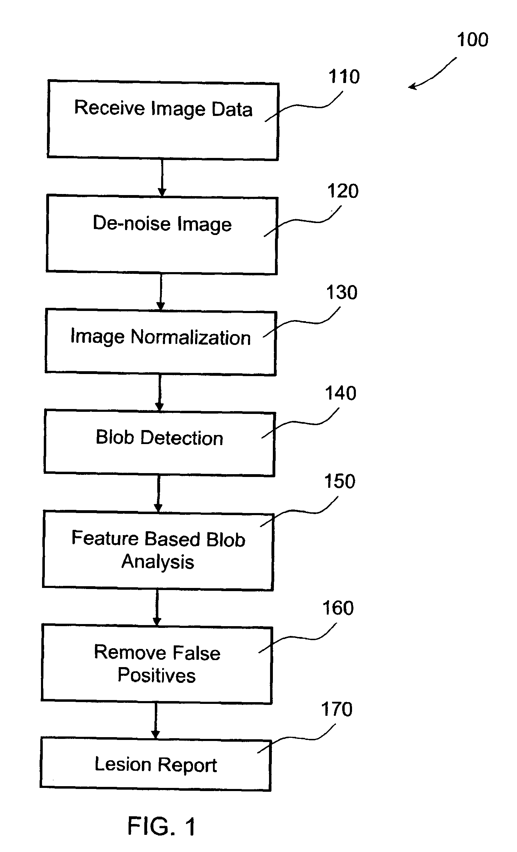

The description which follows and the embodiments described therein are provided by way of illustration of an example, or examples, of particular embodiments of the principles of the present invention. . In the description which follows, like parts are marked throughout the specification and the drawings with the same respective reference numerals.
The present invention is outlined in the claims.
In one embodiment, a sequence of image processing routines are applied to an input image, such as a single breast ultrasound image (or volume data set), to detect and classify each lesion candidate that might require further diagnostic review. Figure **1** is a flow chart that provides an overview of the process **100.**
As a preliminary step **110,** an input image (or volume) is first received for processing. The input image may be retrieved from an image archiving device, such as a Digital Imaging and Communications in Medicine (DICOM) archive, which stores and provides images acquired by imaging systems. The input image also can be directly received from an image acquisition device such as an ultrasound probe. Acquisition parameters can be retrieved together with image data. Acquisition parameters include hardware parameters, such as those due to variations between ultrasound transducer equipment of different vendors, which include depth and transducer frequency, and those operator parameters, such as technologists' equipment or acquisitions settings, examples of which include transducer pressure and time-gain compensation. These hardware and operator parameters can be extracted from data headers as defined in the DICOM standard or transmitted directly with image data when images acquired by imaging systems are processed in real-time.
Subsequent to this preliminary step **110,** the process **100** has a de-noising, i.e., noise-removal or noise-reduction step **120,** to remove or reduce noise from the input image. Noise can be removed or reduced, for example, by applying to the input image an edge preserving diffusion algorithm. Such an algorithm can be used to remove or reduce noise from the image while maintaining and, preferably, enhancing edges of objects in the image.
Next step is to normalize (step **130)** image intensities. As is known to those skilled in the art, intensities of pixels produced by hardware devices of most medical imaging modalities generally suffer from inconsistency introduced by variations in image acquisition hardware. They also suffer from inconsistencies in acquisition techniques applied by hardware operators, such as the gain factor selected by operators during the acquisition of ultrasound images. It is desirable to reduce these inconsistencies. One approach to reducing inconsistencies is to select as a control point a well characterized and common tissue type, for example, the consistently visible subcutaneous fat tissue, and normalize image intensities with respect to the control point. Ideally, intensities are normalized against representative intensities of control point tissues determined or measured dynamically from the input image. Control points, or representative intensities of control point tissues, establish the mapping function from the input tissue type to the output intensities. One example of control points is the computed mean intensity value of subcutaneous fat, which is mapped to the middle point of the output dynamic range. Subcutaneous fat appears consistently below skin-line very near the top of a breast ultrasound (BUS) image. For a BUS image, subcutaneous fat is believed to be a reliable control point tissue for intensity normalization. Other imaged elements, generally selected from but not necessarily limited to organ tissues, such as anechoic cyst, skin tissues, fibroglandular tissues, and calcium, for example, may also be selected as control points. In organs such as prostate or thyroid where significant subcutaneous fat may not always exist, alternative tissue types may be consistently visible for normalization purposes. These alternative control point tissues may be used to account for typical anatomical structures that are generally found in those other imaged organs.

Normalization is a mapping of pixel values from their respective initial values to their normalized values, i.e., converting from their respective initial values to their normalized values according to a mapping relationship between the initial and normalized values. The image can be normalized according to a mapping relationship based on mean fat intensity. A region of the image where the subcutaneous fat is expected to lie is selected and then intensity values of the subcutaneous fat are computed, for example, by applying an intensity clustering algorithm to the selected region. A robust clustering algorithm, such as *k*-means algorithm, may be used to compute an estimated value of the intensity of subcutaneous fat. Other robust clustering algorithms include fuzzy c-means or Expectation-Maximization clustering techniques described in R.O. Duda, "Pattern Classification", John Wiley & Sons Inc., 2001. The clustering algorithm generates a number of clusters, grouped by pixel intensity. A cluster corresponding to subcutaneous fat is identified so that a mean fat intensity, as a representative fat intensity, can be computed from pixel values of pixels in the identified cluster. A mapping relationship may be established for normalizing the input image so that the gray level of fat tissue appears as mid-level grey. This establishes a mapping relationship to convert the representative fat intensity computed by the clustering algorithm. The intensity values of pixels representing other tissues or imaged objects are converted from their respective input values to normalized values according to the mapping relationship. The output of this step is a "fat intensity normalized image". Other control point tissues can be included. Conveniently a mapping from detected intensities of control point tissues to their respective normalized intensities can be established. The mapping relationship, with suitable interpolation, can be used to normalize the image and produce more consistently normalized images. Image intensities of a normalized image provide a more consistent mapping between intensity and tissue echogenicity.

The normalized image is next processed to detect distinct areas of contiguous pixels, or "blobs", that have consistent or similar internal intensity characteristics (step **140).** Different methods of detecting blobs may be employed. In general, one first generates a parameter map, i.e., spatial variation of parameter values at each pixel of the image, for a selected parameter. Then, contiguous pixels having the parameter values satisfying certain criteria, such as exceeding a threshold value, below a threshold value or within a pre-determined range, and forming distinct areas are identified as belonging to blobs, with each distinct area being a detected blob. The selection of parameter is such that the resulting map, in particular, the detected blobs, will aid detection of lesions or other underlying tissue structures. One such map is a density map, based on grey pixel intensity values of the fat normalized image. Blobs in such a map correspond to intensity clusters of pixels, which can be classified into corresponding classes of breast tissue composing the breast, based on their generally accepted relative echogenicity. Other parameters can be selected to generate other parameter maps, as will be described later.

Conveniently, the BI-RADS atlas can be used to classify the echogenicity of a potential lesion as one of several categories:

1. Anechoic: without internal echoes, resembling a dark hole

2. Hypoechoic: defined relative to fat, characterized by low-level echoes throughout the region

3. Isoechoic: having the same echogenicity as fat

4. Hyperechoic: increased echogenicity relative to fat or equal to fibroglandular tissue

5. Complex: containing both hypoechoic (cystic) and echogenic (solid) components

A clustering algorithm can be applied to the density map to cluster pixels based on their grey pixel values. As an example, the resulting clusters could delineate the regions in the density map that correspond to the various BI-RADS categories described above. This process typically generates a large number of clusters or areas of contiguous pixels, i.e., separate image regions, some of whose intensities lie in the range of potential lesions. Each of these regions or shapes is identified as a "density blob".

As noted, in addition to density maps based on grey pixel intensity values, other parameters can be used. One such other parameter is the probability value of a pixel being malignant. For each pixel, a probability value of the pixel being malignant is computed and then assigned to the pixel. This results in a malignancy probability map. Methods of generating a malignancy probability map will be described in detail later. Pixels with malignancy probability above a threshold value, such as 75% or some other suitable value, can be grouped into separate regions. Each of these separated regions is identified as a "malignancy blob".

Not all blobs identified are true lesions. Some of them may be false positive lesion candidates instead of true lesions. To reduce the number of false positive lesion candidates, a feature based analysis of blobs is carried out at step **150.** Details of such a feature based analysis will be given later. Briefly, descriptors of each of the blobs are estimated to quantify each blob's characteristics. These descriptors generally relate to features such as shape, orientation, depth and blob contrast relative to its local background and also the global subcutaneous fat intensity.

The next stage **160** of processing uses the descriptors estimated at step **150** to identify the subtle differences between true lesion candidates that correspond to expert identified lesions and falsely reported candidates. False positives are removed. One approach to differentiating possible true lesions and false positive candidates is to feed the descriptors of each blob through a Classification And Regression Trees (CART) algorithm. The CART algorithm is first trained on a representative set of training images. To train the CART algorithm, blob features extracted or computed from each image of the training images are associated with their respective descriptors and their corresponding expert classifications. At step **160,** the descriptors estimated at step **150** are fed to the trained CART algorithm. The result is an estimated probability that a blob is a lesion, which value is assigned to the blob. Blobs with the estimated probability below a threshold value, i.e., not meeting a pre-determined criteria, are treated as false positives and removed at step **160.** Only remaining blobs are identified and reported at step **170** as lesion candidates for further review and study.

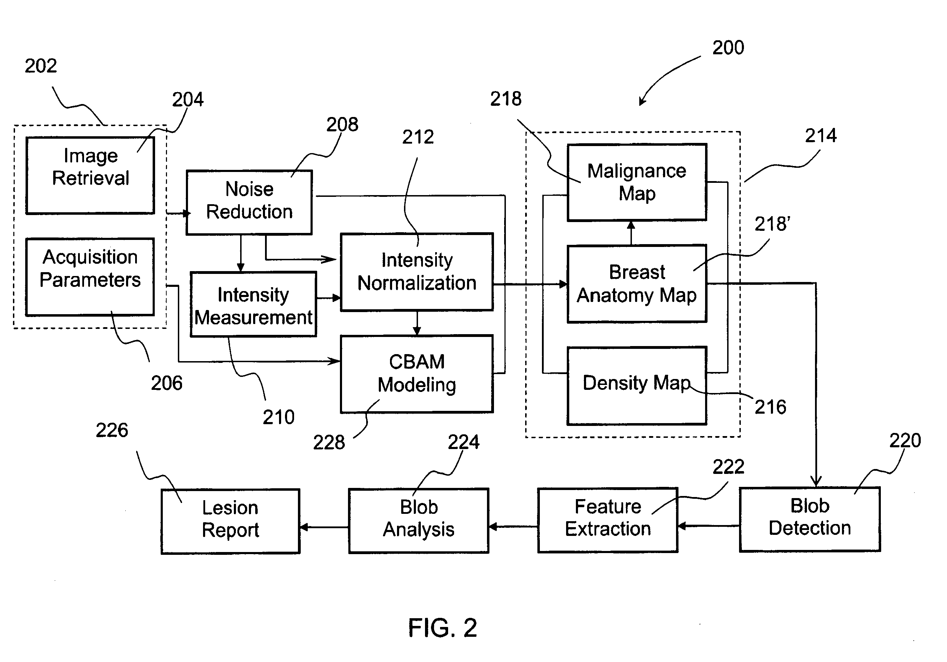

Figure **2** is a schematic diagram showing a CAD system **200** for processing and diagnosing medical images. The CAD system communicates with a source or sources of medical images. The source **202** may be a medical image acquisition system, such as an ultrasound imaging system, from which ultrasound images are acquired in real-time from a patient. The source may also be an image archive, such as a DICOM archive, which stores on a computer readable storage medium or media images acquired by imaging systems. The source may also be image data already retrieved by a physician and stored on a storage medium local to the physician's computer system. An image retrieval unit **204** interfacing with the image source **202** receives the input image data. As will be understood by those skilled in the art, the image retrieval unit **204** may also be an image retrieval function provided by the CAD system **200,** not necessarily residing in any particular module or modules. An acquisition parameters unit **206** extracts acquisition parameters stored in or transmitted together with medical image data. The acquisition parameters unit 206 processes DICOM data and extracts these parameters from DICOM data headers in the image data. It may also be implemented to handle non-standard data format and extract those parameters from image data stored in any proprietary format.

A pre-processing unit, such as a de-noising, or noise reduction unit **208,** may be provided for reducing noise level. Any suitable noise-reduction or removal algorithms may be implemented for this purpose. Image retrieval unit **204** passes received image to the pre-processing unit. The pre-processing unit applies the implemented noise-reduction or removal algorithm to the received image to reduce noise, such as the well recognized speckle-noise artifacts that appear in most US images.

The system **200** includes an intensity measurement unit **210,** or intensity unit. The intensity measurement unit receives an image, such as a noise-reduced image from the noise reduction unit **208,** and measures representative intensities of selected control point tissues, such as mean intensities or median intensities of fat or skin. Different methods may be implemented to measure tissue intensities. For example, a user may identify a region in an image as belonging to a control point tissue, and the intensity measurement unit then evaluates an intensity value for each of the pixels in that user identified region from which to compute a mean intensity of the control point tissue. More sophisticated methods, such as extracting intensity values by way of clustering, may also be utilized. Examples of using *k*-means clustering to compute mean intensity values will be described later.

The system **200** also includes an intensity normalization unit **212,** or normalization unit. The intensity normalization unit **212** normalizes the pixel intensity values of an image based on the representative intensities of control point tissues, so that after normalization, images obtained from different hardware units or by different operators may have a more consistent intensity range for the same type of tissues. The intensity normalization unit **212** generally takes as input a noise-reduced image, pre-processed by noise reduction unit **208,** but can also normalize images forwarded directly from the image retrieval unit **204.** The intensity normalization unit **212** uses output from the intensity measurement unit **210,** i.e., representative intensity values of control point tissues to normalize an image. If only the intensity of fat is factored into the normalization process, the output of the intensity normalization unit is a fat-normalized image. In general, intensities of a set of control point tissues are taken into account by the intensity normalization unit **212** and the result is a general intensity normalized image. Methods employed to normalize an image based on a set of control point tissues will be described in detail later.

The system **200** also includes a map generation module **214,** which includes one or more different map generation units, such as a density map generation unit **216** and a malignancy map generation unit **218.** These map generation units assign to each pixel a parameter value, such as a density value or a malignance probability value. The result is a density map or malignance probability map. The density map unit **216** produces a density map by assigning to each pixel a normalized grey pixel value, i.e., corresponding density value. A malignancy map generation unit **218** assigns to each pixel in an image, usually de-noised and intensity normalized, a probability value of the pixel being malignant, i.e., belonging to a malignant region of the tissue, thus resulting a malignancy probability map. In addition, there can be a breast anatomy map (BAM) unit **218'**. BAM unit **218'** receives an input medical image, such as a normalized image or a de-noised image, and categorizes each pixel of the image with possible tissue types. The image having its pixels classified can be further processed by the malignancy map generation unit **218.** A probability value of a pixel being malignant can be assigned to each pixel, which will also result in a malignancy map. Processes that can be implemented in these map generation units will be described in detail later.

These maps are clustered into blobs. A blob detection unit **220** is provided to cluster pixels in a map and to detect a region of interest (ROI) enclosing each of the blobs ("blob ROI"). A blob can be detected by clustering, or by grouping pixels having a parameter satisfying a pre-selected criteria, as noted earlier. By tracing boundaries of the blobs or otherwise determining the boundaries, the blob detection unit **220** also demarcates the blobs that it has detected. A feature extraction unit **222** is provided for extracting features or characteristics from the detected blobs. Different categories of features, or descriptors, may be defined and classified. For example, there can be features related to shape of blobs, to grey level variations, or to spatial location of a blob relative to anatomic structure in the imaged region. The feature extraction unit **222** is implemented to extract, i.e., to detect and/or compute, features or descriptors according to each defined category of features. Of course, with more categories of features defined, the functionality of the feature extraction unit 222 can be expanded to handle the expanded range of features.

Detected blobs are further analyzed. For example, morphological features of a blob, such as shape, compactness, elongation, etc., can be computed and analyzed as will be described in greater detail later. Prior to the analysis, the blob may undergo morphological modifications, such as filling in any "holes" within the blob ROI, or smoothing the bounding contour of the ROI using morphological filtering. These blobs are analyzed by a blob analysis unit **224,** taking into account features extracted and numerical values assigned to each of the features where applicable. The result of this analysis is combined to compute an estimated likelihood value that the blob is likely malignant, i.e., a true lesion. The blob analysis unit **224** also assigns the estimated likelihood value to the blob, once the value being computed or otherwise determined. All blobs having likelihood values above a predefined threshold value can be reported for further study by a radiologist, or be subject to further automated diagnostic evaluation. Identification of lesion candidates (or suspect blobs) to report and reporting of these lesion candidates are carried out by a lesion report unit **226.**

As a further improvement, the system **200** also can include a coarse breast anatomy map (CBAM) modeling unit **228.** Briefly, as will be described in details later, a CBAM model is a layered model of breast, which divides a breast image into a number of primary layers, to match the general anatomical structure of a breast. CBAM modeling provides an automated approach to estimating locations of primary layers, such as subcutaneous fat or mammary zone. Estimated locations of boundary surfaces can be used by, for example, intensity detection unit **210** for estimating fat intensity, or by BAM unit **218'** to classify only pixels in the mammary zone. Details of a process that can be implemented in the CBAM modeling unit **228** will be described later.

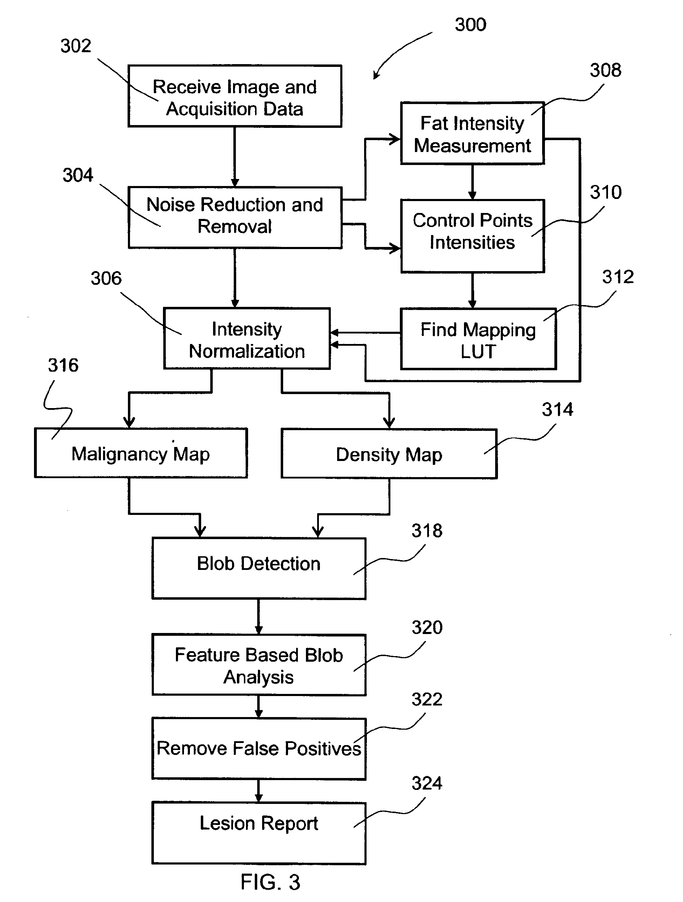

Referring to Figure **3,** there is shown a process of automatically segmenting a medical image, such as an ultrasound image, and classifying the segmented masses detected in the medical image into lesion candidates and false positives. This method may be implemented using the CAD system illustrated in Figure **2** or as part of a CAD and image acquisition system embedded in image acquisition hardware, among other possibilities.

The first step is to receive input image data, which includes a medical image data and its associated image acquisition parameters (step **302**). This may be carried out by the image retrieval unit **204** and the acquisition unit **206,** for example. Next, an input medical image is pre-processed to reduce noise (step **304).** To reduce the typical high level of noise in ultrasound input images, this step generally includes noise reduction and removal. A de-noising technique should not only reduce the noise, but do so without blurring or changing the location of image edges. For example, the input image can be enhanced by an edge preserving diffusion algorithm that removes noise from the ultrasound image while maintaining and enhancing image edges, ensuring that they remain well localized. Such a de-noising step therefore may achieve the purposes of noise removal, image smoothing and edge enhancing at the same time. A noise reduction unit **208** (see

Figure 2) may implement any suitable de-noising, i.e., noise reduction and removal algorithm for carrying out the de-noising step. Preferably, a noise-reduction or removal algorithm is selected with a view to enhancing edges of features captured in the image, without blurring or changing the location of image edges. Furthermore, the edge enhancement or noise-removal process is configured as a function of the image acquisition parameters, to account for the inherent differences in the image characteristics due to operator settings, or hardware filtering.

While many different suitable edge preserving image filtering algorithms may be used, the following describes the use of a non-linear diffusion method, with the understanding that this is not the only method suitable or available. Non-linear diffusion method is a well-known image processing enhancement technique that is often used to remove irrelevant or false details in an input image while preserving edges of objects of interest. Non-linear diffusion smoothing is a selective filtering that encourages intra-region smoothing in preference to inter-region smoothing, preserving the sharpness of the edges. The method consists of iteratively solving a non-linear partial differential equations:

$$\frac{\partial I}{\partial t} = \nabla \bullet \left[ C \nabla I \right] \qquad (1)$$

where $I$ denotes the input image, t represents time and C is a conductivity function dependent on the gradient norm $\|\nabla I\|$. A simple example of the conductivity function C has the form:

$$C\left(\|\nabla I\|\right) = e^{-\frac{\|\nabla I\|^2}{k^2}}$$

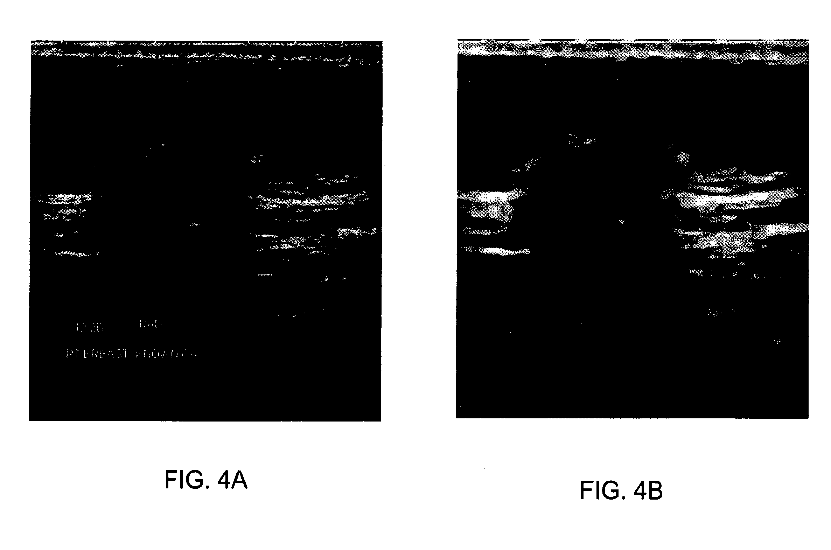

where $k$ plays the role of contrast parameter, i.e., structure with gradient values larger than $k$ are regarded as edges, where diffusivity is close to 0, while structures with gradient values less than $k$ are considered to belong to interior regions. The algorithm is described in Weickert, J., "Anisotropic Diffusion in Image Processing", ECMI Series, Verlag, 1998. An example of the application of edge preserving diffusion is shown in Figure **4** in which Figure **4a** shows an input image before the application of a de-noising algorithm and Figure **4b** shows a smoothed image.

To simplify lesion candidate detection, the input image $I$ is normalized to ensure a consistent mapping between image pixel intensity and tissue echogenicity, mitigating the variability of gain factor settings between images. A normalization step **306** is applied to the de-noised image. The normalized pixel values have more consistent ranges of pixel values for tissues represented in the images. The echogenicity of subcutaneous fat is preferably represented by a mid-level grey intensity in the normalized image. For typical 8-bit ultrasound images, the mid-point of intensity value corresponds to an intensity of 127, in a range of grey levels between 0 and 255. In order to apply intensity normalization, the intensity of fat is detected first at step **308**. The result of the normalization step **306** is a fat-normalized image. In a more general approach, in addition to subcutaneous fat, intensities of a number of control point tissues are measured or detected (step **310),** for example, by the intensity measurement unit **210**. The mapping relationship may be represented by a mapping look-up table (LUT). The mapping LUT is computed from representative intensities of control point tissues and their respective assigned values (step **312).** The image is next normalized (step **306)** according to the mapping LUT. In the following, the fat-based normalization is described first.

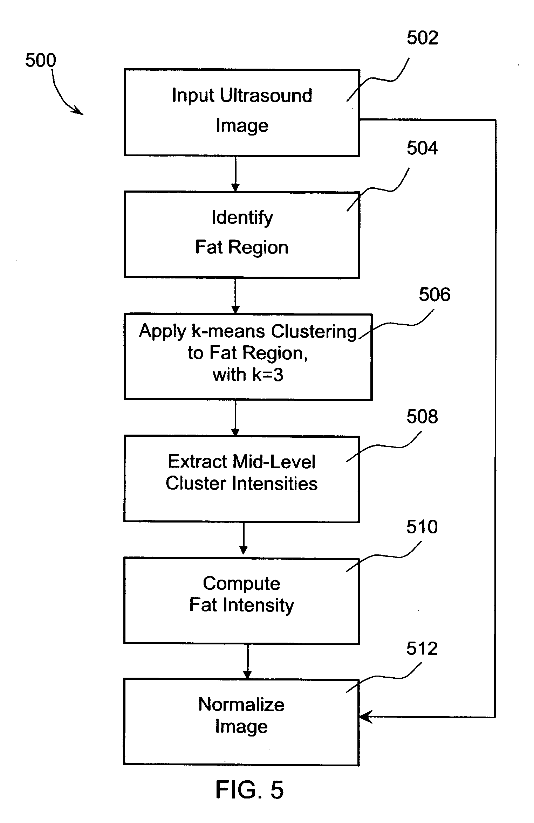

Figure **5** illustrates an automated process **500** for determining intensities of subcutaneous fat and then using its mean intensity as a representative value of fat intensity to normalize an original input image. Ultrasound image acquisition protocols generally encourage sonographers to configure time-gain compensation setting to ensure a uniform mapping of echogenicity to intensity, to facilitate interpretation. This permits the assumption that spatial variability of the mapping is minimal, although it will be understood that further refinement to the method described herein may be made to take into account any detected spatial variability.

The method starts by receiving an original input image, such as an ultrasound input image (step **502).** Next is to select or identify an ROI (step **504)** in the input image that is primarily composed of subcutaneous fat pixels. Empirically, typical depth of the anterior surface of the subcutaneous fat region is approximately 1.5mm and typical depth of the posterior surface of the subcutaneous fat region is approximately 6mm. Despite variation in precise locations of the subcutaneous fat region, a significant portion of the image region between the depths of 1.5mm and 6mm tends to be composed of fat.

The fat region may be selected by cropping the de-noised image between the target depths. An ROI is thus obtained to represent the fat region in the original input image. In some areas, such as around the nipple area, the subcutaneous fat region is more posterior than the more typical location at the very top of the image. The selection of fat region may be further refined by detecting the presence of nipple, nipple pad or other features in the image area, and where necessary, shifting or changing the contour of estimated subcutaneous fat image strip location to accommodate these cases. Other methods, such as modeling depths of tissue types in breast ultrasound images, may also be employed to delineate boundaries of the subcutaneous fat region. One such modeling method, a so-called CBAM method, will be described in detail shortly.

Next, at step **506,** a robust intensity clustering algorithm, such as a $k$-means clustering algorithm, is applied to the intensities in the subcutaneous fat region. Although the use of $k$-means algorithm is described here, as noted, other robust clustering algorithms such as fuzzy c-means or Expectation-Maximization clustering techniques can be used in place of $k$-means algorithm. The $k$-means clustering algorithm, where $k=3$, is configured to divide the fat region, such as subcutaneous fat image strip, into three clusters of pixel intensities: anechoic and hypoechoic regions of the strip are identified by the lowest pixel intensity cluster, isoechoic regions of the strip are identified by the mid-level intensity cluster, and finally, hyperechoic regions are indicated by the high intensity cluster.

At step **508,** intensities of the mid-level intensity cluster are computed or extracted. A representative intensity, or mean fat intensity in this example, is computed at step **510** from the extracted intensities. The result of this clustering operation is a robust estimate of the intensity of fat in the image strip. As fat tissues are expected to have the same intensity, whether the fat tissues are located within the subcutaneous strip or elsewhere, this estimate can be used as a representative intensity of fat throughout the image. Finally, at step **512,** the original input image received at step **502** is normalized using the estimated fat intensity, resulting in a fat-normalized image. The normalization process will be further described in detail in this document.

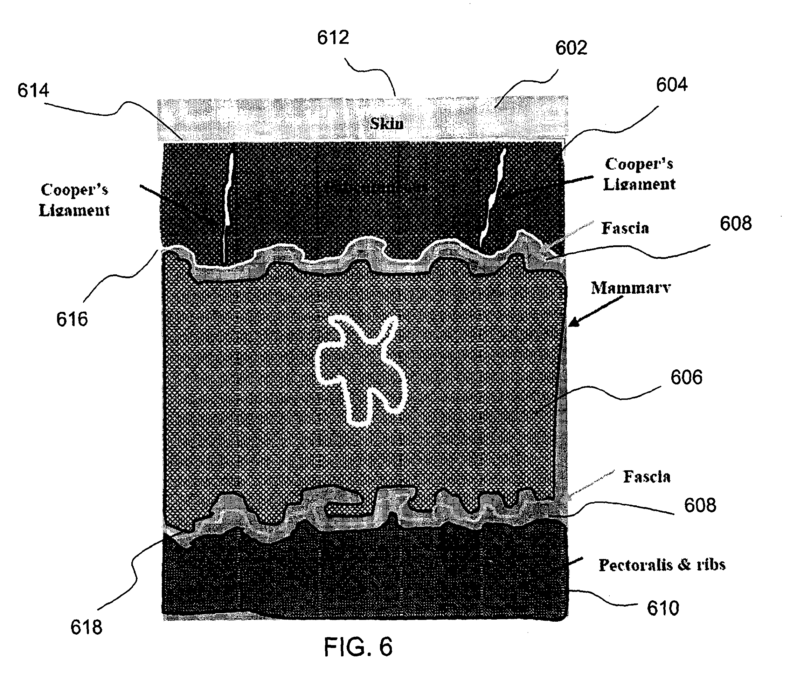

As indicated earlier, the region of subcutaneous fat may also be identified through modeling the depth of various tissue types in a breast ultrasound image. Figure **6** illustrates the typical structure of a breast ultrasound image, which includes four primary layers. The skin layer **602** appears as a bright horizontal region near the top of the image followed by a uniform region of subcutaneous fat **604** (often in the shape of a horizontal image strip) that is separated from the glandular region or mammary zone **606** by retro-mammary fascia **608.** At the bottom of the image is retro-mammary zone **610,** i.e., chest wall area, typically pectoralis and ribs, represented as dark regions and separated from mammary region **606** by fascia **608,** again.

Skin line **612,** i.e., outer surface of skin layer **602,** provides a robust reference surface for measuring depths of various primary layers. The depth of each primary layer's start and end may be conveniently measured by distance from skin line **612** to a boundary surface between the primary layer and its neighboring primary layer. For example, the depth of a first boundary surface **614** separating skin layer **602** and subcutaneous fat **604** provides a measurement of thickness of skin **602.** Similarly, the thickness of subcutaneous fat **604** can be obtained by measuring the depth of a second boundary surface **616** between subcutaneous fat **604** and mammary region **606** and calculating the difference of depths of the first and second boundary surfaces **614, 616.** When the depth of a third boundary surface **618** between mammary region **606** and retro-mammary zone **610** is also known, the thickness of mammary zone **606** can be computed from the difference of depths of the second and third boundary surfaces **616, 618.**

As is known, the thickness of primary layers varies across a breast and with the size of a breast. Advantageously, a coarse breast anatomy map (CBAM) model can be established to model, i.e., to estimate, the approximate locations of the primary layers in a breast (Figure **6).** Locations of primary layers can be indicated by boundary surfaces separating the neighboring layers. A CBAM model represents locations of boundary surfaces using a set of boundary surface look-up tables (LUTs). To take into account size variation of breast, a parameter reflecting the size of a breast is selected to parameterize different sets of boundary surface LUTs, hence the parameterized CBAM model. One such size parameter may be the maximum depth of a boundary surface measured from skin surface.

In the following, maximum mammary zone depth, $MZD_{max}$, is used as a size parameter to illustrate the creation, calibration and application of a parameterized, layered model of breast tissues. Mammary zone depth, MZD, measures the depth of the mammary zone from skin, i.e., the distance between skin line **612** and third boundary surface **618** that separates mammary region **606** from retro-mammary zone **610.** Typically, $MZD_{max}$ occurs in a central region of a breast, a region often coincided with the location of nipple, which is a distinct anatomical feature. It will be understood that any other suitable size parameters reflecting the size of an imaged breast may be selected for modeling purposes.

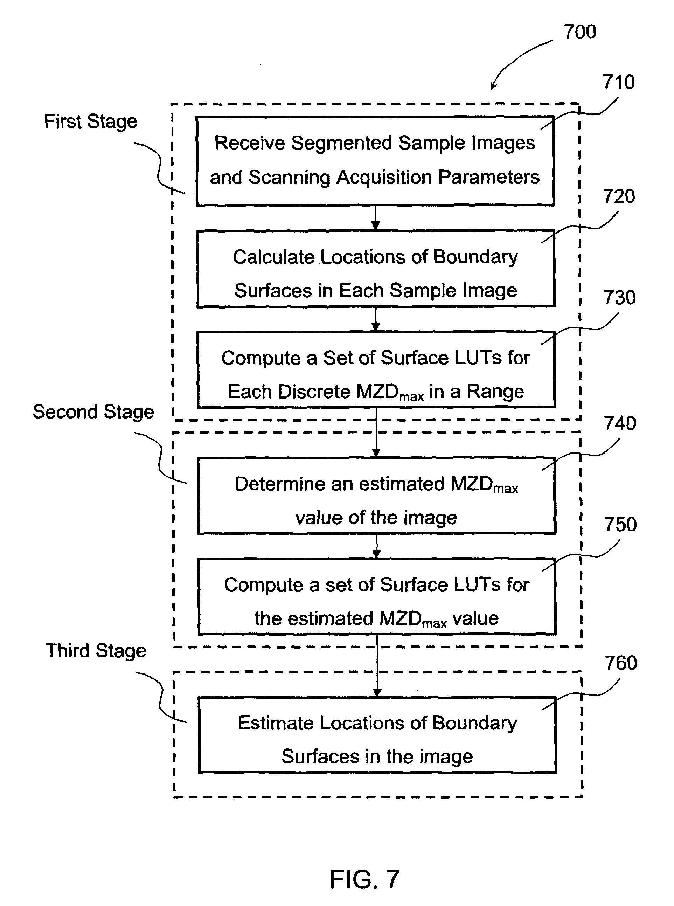

Figure 7 shows a flow chart of a process **700** of establishing a parameterized model of the primary tissue layers in a breast, and estimating locations of the primary tissue layers, namely the depths of skin, subcutaneous fat, mammary tissue and retro-mammary tissue layers in a BUS image. It should be noted that this process is equally applicable to two-dimensional (2D) and three-dimensional (3D) breast ultrasound images.

The process **700** broadly includes three stages. The first stage is to construct a parameterized model of primary layers. The parameterized model is constructed from a large number of input images and generated over a selected range of the size parameter. Second, upon receiving a new BUS data set, the value of the model's size parameter, $MZD_{max}$ is estimated or determined from acquisition parameters. The estimated value of the size parameter for the new BUS data set is passed to the parameterized model to dynamically generate a new CBAM model for the new BUS image. Third, locations of boundary surfaces of the primary layers are computed from the new CBAM model. At each of these stages, steps within each of the stages, and some of their variations are now described in detail below.

The first stage is to construct the model, e.g., by generating a representation of physical breast anatomy by dividing a breast into four primary layers and then computing estimated locations of the four primary layers by training, i.e., calibrating, the parameterized model on a large number of sample BUS images. Each of the sample BUS images is manually segmented, i.e., having the boundary surfaces between the primary layers marked by an expert. The model is calibrated also for a large number of size parameter values, i.e., maximum zone depth values, in order to span the entire thickness domain in a breast (for example, between 2 cm and 5 cm).

Referring to Figure 7, at the first step **710,** a large number of sample BUS image data are retrieved, each sample image being manually segmented. Along with each sample image, also received are scanning acquisition parameters such as pixel size, transducer angle, maximum mammary zone depths value $MZD_{max}$ and position of transducer on breast on a breast clock map. Next, at step **720,** locations of each boundary surfaces are calculated for each segmented image using the scanning acquisition parameters. For example, pixel size parameter may be used to convert between depth values and pixel values. Similarly, transducer scanning angle can be used (when available) to recalculate depth values based on triangle geometry whenever the transducer orientation is not perpendicular to the skin surface. Next, at step **730,** for each boundary surface, a large number of surface look-up tables (LUTs) are generated, each LUT corresponding to an $MZD_{max}$ value, or a bin of $MZD_{max}$ values of the training BUS images. The surface LUTs allow one to compute location of boundary surfaces. Each surface LUT (and the corresponding boundary surface) is a function of position on a breast, such as that measured with reference to the nipple. The nipple is often the position where the highest value of MZD occurs, $MZD_{max}$.

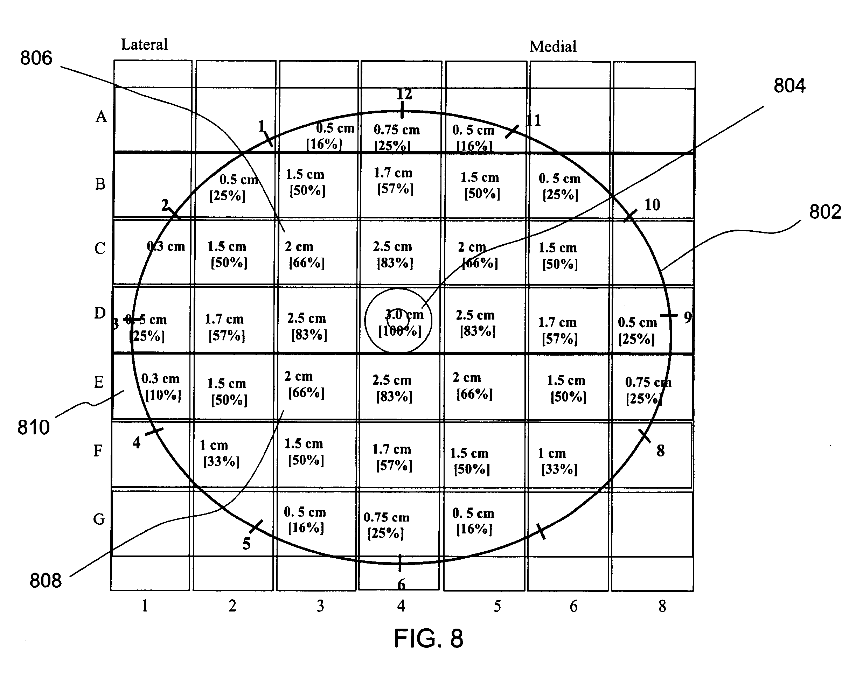

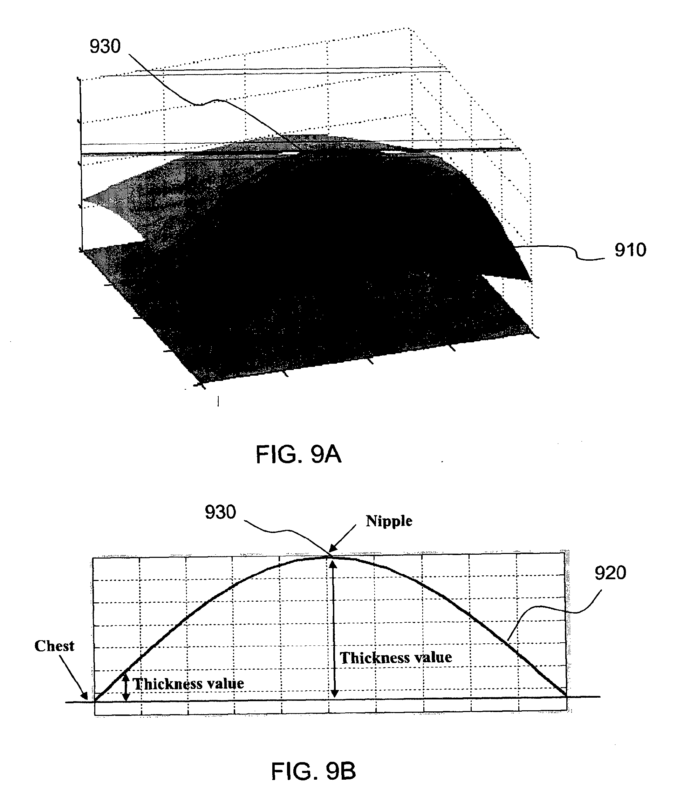

Figures **8** and **9** are some examples of an MZD surface, namely, the third boundary surface **618,** which is the boundary surface between mammary zone **606** and retro-mammary fascia **608.** Figure **8** shows an example of variations of MZD values with respect to position on a breast clock map **802,** starting from the nipple **804,** where the thickest layer of mammary zone tissue typically occurs. The example shows a central region **804** with an $MZD_{max}$ value of 3 cm, coinciding with the nipple position. The MZD value at the nipple position is thus indicated to be 100% of the $MZD_{max}$ parameter. A more distant cell **810** at a larger distance from the nipple position **804** has a smaller MZD value. The example shows a generally symmetric MZD surface. For example, cells **806, 808** at about equal distance to the central region **804** have about the same MZD value but in practice, the geometry of the surface is often asymmetric. A 3D view of the MZD surface **910** is shown in Figure **9a,** while a 2D profile **920** is presented in Figure **9b.** They both demonstrate the general trend of decreasing MZD value at larger distance from the point of $MZD_{max}$, or the nipple position **930** and the general symmetric property about the point of $MZD_{max}$. As noted, a surface LUT is created for the MZD surface. Similarly, other primary layer boundaries, e.g., boundary surfaces between skin **602** and subcutaneous fat **604,** then between subcutaneous fat **604** and mammary zone **606,** like that shown in Figs. 8 and 9 can be calculated and similar LUTs can be generated for these boundary surfaces.

These 3D (or 2D) LUTs for discrete $MZD_{max}$ values are stored. They are subsequently exploited when a new set of LUTs is computed for a new breast US scan, i.e., for a new $MZD_{max}$ value. The pixel size parameter may then be used to scale the MZD values to pixel values, while the transducer scanning angle can be used to recalculate MZD values based on triangle geometry wherever the transducer is not perpendicular to the skin surface.

The next stage is to generate a new coarse breast anatomic map (CBAM) model for the new breast US image, i.e., the medical image received at step **302.** Referring to Figure 7, the image data and the associated scanning acquisition parameters are retrieved at step 740, or if already received at step **302,** simply forwarded to CBAM module **228** for processing. As noted, a received image has associated therewith an estimated $MZD_{max}$ value. A set of surface LUTs, for the received medical image's $MZD_{max}$ value, is computed at step 750. Conveniently, if the estimated $MZD_{max}$ value of the new BUS image matches one of the discrete $MZD_{max}$ values used in generating and calibrating the parameterized layered model, the corresponding surface LUTs are simply retrieved and can be used directly in the subsequent steps. Otherwise, a new set of surface LUTs corresponding to the image's $MZD_{max}$ value must be computed from the parameterized layered model. One approach is to simply select two sets of surface LUTs, whose $MZD_{max}$ values bracket or are the closest to the image's $MZD_{max}$ value and then compute the new set of surface LUTs from these two sets of surface LUTs by interpolation or a simple weighted arithmetic average between these two models. If the particular $MZD_{max}$ is not bracketed by two surfaces in the model, then the new set of surface LUTs may be extrapolated from the model with the most similar $MZD_{max}$ value. Of course, a more refined approach, using more than two sets of LUTs, also can be used to compute the new set of surface LUTs. The new set of computed surface LUTs constitutes the new CBAM model.

Once the new set of LUTs is computed, the final stage is to compute estimated boundary surfaces, i.e., locations of the primary tissue layers using the new set of computed LUTs (step **760).** Where necessary, scanning acquisition parameters can be used to correct, i.e., to compensate for, variations introduced by different scanning parameters.

As this process takes advantage of CBAM models, it is referenced herein as CBAM method **700.** While the CBAM method may have general applications e.g. estimating locations of primary layers in any BUS image, one application is to identify subcutaneous fat region **604.** Pixels between first boundary surface **614** and second boundary surface **616** (see Figure **6)** are considered to consist primarily of fat tissues. A mean fat intensity can be extracted from intensities of these pixels, as described earlier, either by applying a k-clustering technique, or by simple averaging, among others.

It will be understood that the CBAM method has some general applications. For example, grey level intensities tend to vary significantly from primary layer to primary layer. For visualizing an imaged breast, each layer identified from a CBAM model can be rendered individually, thereby alleviating the difficulty caused by greatly different intensities. Alternatively, when rendering the image for visualization, only a portion of the imaged breast is rendered. For example, the skin layer or retro-mammary layer, or both, may be excluded from the rendering of the mammary layer. Additionally, as will be described below, further processing and identification of lesions can take advantage of knowledge of locations of different primary layers, by limiting application of filters to particular primary layer or layers where certain types of tissues or lesions are more likely to occur and the filters are designed to detect these types of tissues or lesions.

Having identified the image region corresponding to the subcutaneous fat (for example, using the CBAM method), and estimated a representative fat intensity, we can return to Figure **3** where the representative fat intensity is used to normalize the input image at step **306.** The normalization step maps the input range [*MinGreyLevel, MaxGreyLevel*] of the input image to a dynamic range that spans from 0 to $2^{NrBits}$- 1, where *NrBits* is the number of bits per pixel of an output image. The estimated intensity of subcutaneous fat is mapped to a point generally near the mid-level of intensities, such as the middle intensity of the output dynamic range ($2^{NrBits}$ - 1)/2, e.g., 127 for *NrBits=8.* It will be understood that the normalization is not limited to *NrBits=8,* which is only an example.

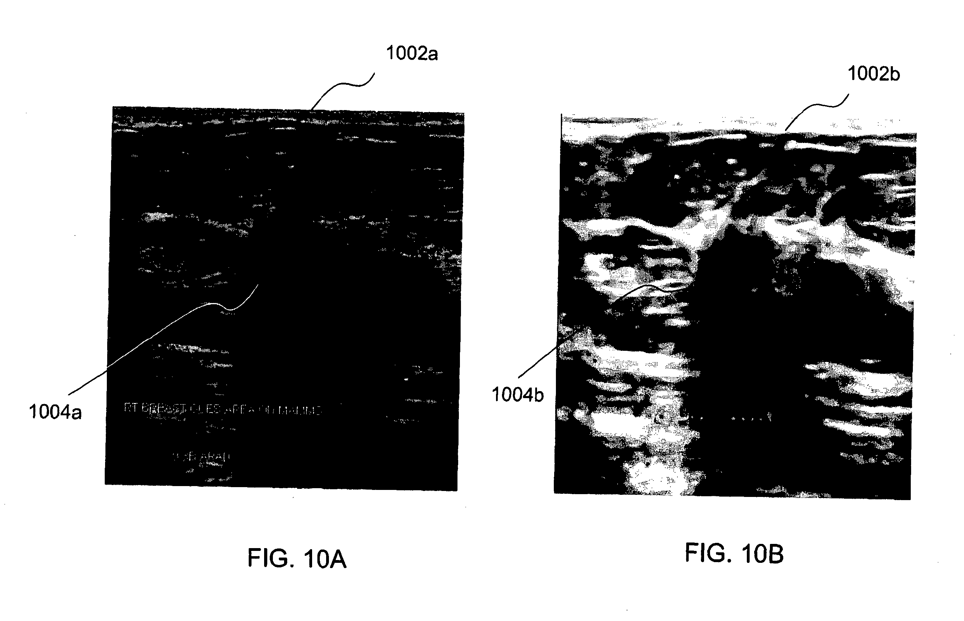

In Figure **10,** examples of an ultrasound image are shown before (Fig. **10a**) and after (Fig. **10b**) intensity normalization and image smoothing, illustrating a mapping of the subcutaneous fat zone **(1002a** and **1002b)** in the top edge region of the image to a mid-level grey. Hypoechoic and anechoic regions are more easily identified in the normalized image. As lesions very often fall in one of these two echogenicity categories, the normalization process facilitates their detection and delineation.

After the intensities of the image are normalized, the next stage is directed to lesion candidate blob detection. Referring to Figure 3, to accommodate automated lesion candidate detection to various types of tissue echogenicity, a parameter map is first generated. The following assumes the generation of a density map at step **314,** i.e., the step generates a density map using normalized grey pixel values. Generation and processing of other types of parameter maps will be described later. Normalized grey pixel values can be clustered to a number of echogenicity ranges for breast tissues that mimic the tissue composition of the breast. The regions that belong to any one of the anechoic, hypoechoic, or isoechoic classes are tracked and stored individually as potential lesion candidates, which may undergo further analysis to assist their classification, as will be described below.

To detect blobs (step **318)** from a density map, the density map is first clustered to generate a clustered density map. The computation of a clustered density map from a density map may consist of a robust clustering or applying a classification algorithm to the intensities of the normalized image to detect the anechoic, hypoechoic and isoechoic regions in the image. Following this approach, pixels in an input image are first grouped into five categories of regions based on pixel intensity. In general, the first cluster includes the anechoic dark regions of the image. The second cluster captures the hypoechoic regions in the BUS. The third cluster includes the isoechoic fat areas. The fourth cluster contains the slightly hyperechoic glandular areas, and finally, the skin, Cooper's ligaments, speckle noise and microcalcifications compose the hyperechoic fifth cluster. To cluster the pixels, *k*-means clustering algorithm with a value *k*=5 is applied to the normalized image. This partitions the dynamic range of the normalized input image into five intensity clusters, corresponding to the five categories listed above. Each contiguous region in a cluster tends to have consistent or similar interior intensity characteristics and is therefore a "density blob". Each of these contiguous, distinct regions can be analyzed individually as a potential lesion. Detecting blobs then only requires generating outline contours of the clustered, contiguous regions, i.e., density blobs.

To differentiate possible true lesions from other types of masses seen in a medical image, different features or characteristics associated with a blob are extracted and analyzed to classify the blob (step 320). Generally, these features or characteristics are referred to as descriptors as they are descriptive of what the blobs may represent. The following describes one approach to a feature-based blob analysis. The analysis starts by first selecting a set of pertinent descriptors. These descriptors are next analyzed to assess their relevancy to an estimated probability that the blob may be malignant. A CART tree, for example, can be employed to assess these descriptors and to produce an estimated probability that the blob may be malignant. A value representing likelihood the blob of being malignant is then computed and assigned to the blob. Blobs with a high estimated probability are marked as likely lesions. Each of these steps is described in detail below.

As mentioned earlier, these features, or descriptors, are inputs to a CART operation. A CART algorithm is first developed by analyzing these blob descriptors and their corresponding expert classifications for a representative set of training images. The CART algorithm is then applied to the set of descriptors of each blob identified from the input image. The output of the CART tree operation is utilized to obtain a likelihood value, or estimated probability, of a blob being a lesion. False positives are removed (step 322) based on likelihood values assigned to the analyzed blobs.

To prepare for the CART operation, first, a number of blob features are defined to differentiate between solid nodules and non-suspicious candidates. The features or descriptors can be classified into three main categories: shape, grey level variation and spatial location. Each category of descriptors can be further divided into subclasses. For example, shape descriptors can be split into two subclasses: features generated from the segmented blob candidate and features generated as result of fitting an ellipse to the blob's contour. Compactness indicator and elongation value belong to the first subclass. Compactness indicator can be calculated as follows:

$$Compactness = 4\pi \, \frac{BlobArea}{BlobPerimeter^{2}}$$

where a circle has a compactness of 1 while a square has a compactness value of $\dfrac{\pi}{4}$. In the expression above, *BlobArea* is the area of a blob and *BlobPerimeter* is the total length of a blob's outline contour.

Elongation indicator is defined using a width to height ratio and can be calculated as follows:

$$Elongation = \frac{WidthBlobBoundingBox}{HeightBlobBoundingBox}$$

In the expression above, *WidthBlobBoundingBox* is the width of a rectangular box that tightly bounds the blob and *HeightBlobBoundingBox* is the height of the rectangular bounding box. The elongation values are always greater than zero. A value of 1 describes an object that is roughly square or circular. As the elongation value tends to infinity, the object becomes more horizontally elongated, while the object becomes more vertically elongated as its elongation value approaches zero.

Similarly, features generated as a result of fitting an ellipse to the blob's contour also can be further divided into two subclasses: eccentricity and orientation of major axis. *Eccentricity* is defined as:

$$Eccentricity = \frac{ShortEllipseAxisLength}{LongEllipseAxisLength}$$

In the expression above, *ShortEllipseAxisLength* is the length of the short axis of the fitted ellipse and *LongEllipseAxisLength* is the length of the long axis of the fitted ellipse. The eccentricity values are strictly greater than zero, and less than or equal to 1.

Orientation of major axis is computed from:

$$MajorAxisOrientation = \frac{atan\left(\dfrac{2\mu_{11}}{\mu_{20} - \mu_{02}}\right)}{2}$$

where $\mu_{11}$, $\mu_{20}$, $\mu_{02}$ are second order moments that measure how dispersed the pixels in an object are from the center of mass. More generally, central moments $\mu_{mn}$ are defined as follows:

$$\mu_{mn} = \sum_{x=0}^{columns} \sum_{y=0}^{rows} \left(x - x_{mean}\right)^m \left(y - y_{mean}\right)^n; \quad for\ m+n > 1$$

where $(x_{mean}, y_{mean})$ is the coordinate of the center of mass.

The grey level variation descriptors are also split into two categories: a category describing grey level variation within a lesion and a category describing lesion grey level contrast relative to the lesion's local and global background. Features that describe grey level variation of pixels inside a lesion can be further divided and grouped into the following four subcategories:

    a. Variance, which is a second order central moment:

$$Variance = \frac{1}{N-1}\sum\nolimits_{i=1}^{N}(x_i - \mu)^2$$

where the blob contains N pixels, and the gray level of the $i^{th}$ pixel within the blob is represented by $x_i$, while $\mu$ is the mean gray level of all the pixels inside the blob.

b. Skewness, which is the third order moment and describes asymmetry in a random variable:

$$Skewness = \frac{1}{N-1}\frac{\sum_{i=1}^{N}(x_i - \mu)^3}{\sigma^3}$$

where $\sigma$ is the standard deviation, or the square root of the variance. Negative values for the skewness indicate data that are skewed left and positive values for the skewness indicate data that are skewed right.

c. Kurtosis, which is the forth moment:

$$Kurtosis = \frac{1}{N-1}\frac{\sum_{i=1}^{N}(x_i - \mu)^4}{\sigma^4}$$

Positive kurtosis indicates a "peaked" distribution and negative kurtosis indicates a "flat" distribution.

d. $L_2$ gradient norm of all pixels contained in the segmented lesion:

$$GradientNorm = \sqrt{\sum_{i=1}^{N}\nabla x_i^{\,2}}$$

Features that describe variation of grey level of pixels inside the lesion relative to its background and the subcutaneous fat region can be grouped into the following two subcategories:

a. Visibility of the lesion on given background. The background is defined as a region that begins at the outer edge of the blob's bounding contour, and extends a number of pixels beyond the contour. One way to delineate such a background region surrounding a blob area is to apply a morphological dilation filter to a binary image that indicates the blob area, then subtract the original blob area from the dilated area, to leave a tube-like background region that directly surrounds the blob area. An even simpler background area might be derived by padding a rectangular box that bounds the blob area, and considering all the pixels within the padded rectangle, but outside the blob area, to represent the background area. A visibility indicator can be computed from the following:

$$Visibility = \frac{MeanLesion - MeanBackground}{MeanLesion + MeanBackground}$$

In this expression, *MeanLesion* is the mean grey value of pixels inside the lesion and *MeanBackground* is the mean grey value of pixels of the background region(s).

b. Normalized value of the mean grey pixel value of pixels inside the lesion and fat value from the subcutaneous region:

$$MeanLesion2SubcutaneousFat = \frac{MeanLesion - SubcutaneousFat}{SubcutaneousFat}$$

After an analysis of features computed or extracted from the image as described above and with false positives removed, at a final step (step **324),** all blobs having likelihood values above a threshold value are reported, for example, by showing them on a display device, for further study by a radiologist, or forwarded to additional CAD modules for further automated analysis. They may be reported after sorting, so that they can be presented in order of descending likelihood. This completes the process of automated lesion detection.

Variations can be introduced to the process described above to improve performance of automated detection of lesion candidates. For example, the above description generally relates to the processing of a fat-normalized image. In general, normalization of input images may include several control point tissues, instead of only subcutaneous fat. This requires establishing a number of reference intensity values ("control point values") in the input image intensity range, in addition to the intensity of fat. Each control point comprises two values, a representative intensity of the control point tissue as measured from the input or de-noised image and an expected or assigned intensity of the control point tissue. The intensity of fat determined at step **308** (a step described in great detail as process **500)** and intensities of other control points are used to prepare an image normalization lookup table.

In one embodiment, to calculate the control point values for the normalization LUT, a robust clustering operation (e.g. *k*-means clustering) is applied at step 310 (see Figure 3) to the entire image instead of subcutaneous fat region only. The fat and hyperechoic intensity values obtained in the first clustering run are used to configure the subsequent cluster centers for fat and skin while classifying the entire image. The result of this second clustering operation is the estimation of several additional distinct cluster centers to capture the intensities that represent anechoic, hypoechoic and hyperechoic echogenicities. These broad classes (e.g., hyperechoic) often contain several subclasses of echogenicity that may also need to be distinctly detected by clustering. Examples include similar but statistically distinct echogenicities of skin and fibroglandular tissue. To reduce the level of speckle noise, the smoothed image can be used. Alternatively, a median filter may be applied to the retrieved image before the clustering algorithm is applied.

After intensities of all control points are estimated or measured, a consistent mapping between intensities of pixels in the pre-processed image and a resulting image needs to be established. The mapping relationship (step **312)** can be established in a variety of manners. For example, the control points can be mapped to pre-determined values or assigned values, with pixel values between neighboring control points interpolated. Pixel values with intensities between the minimum or maximum pixel value and its neighboring control point can also be interpolated. For example, a smooth curve can be fitted to these control points to interpolate the values between them, including the minimum and maximum pixel values, using any known curve fitting method, such as spline fitting.

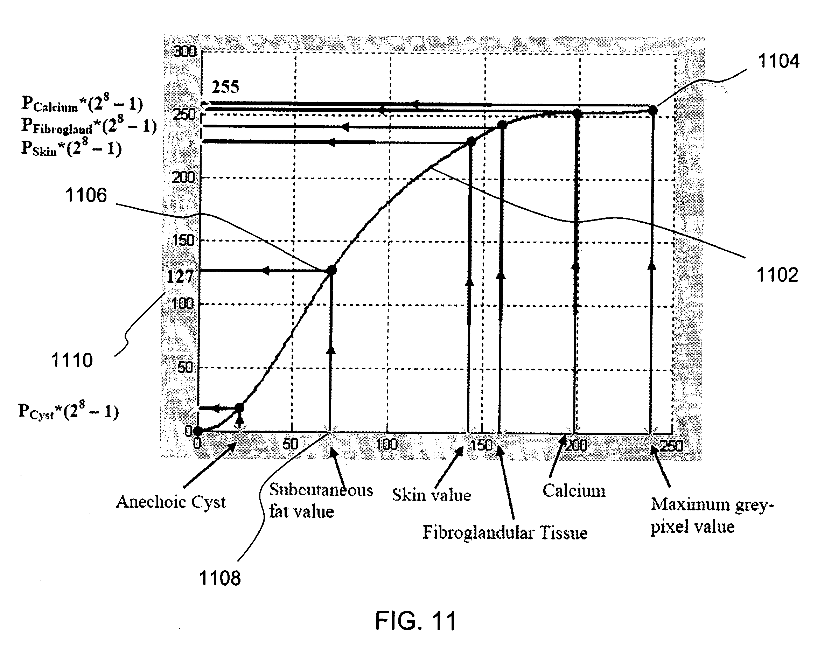

Figure **11** illustrates the mapping of measured control point tissues to their respective assigned grey pixel intensity values. In this embodiment, a smooth curve **1102** connecting these control points **1104** (one of them being fat **1106)** is first found. For example, spline interpolation takes as input a number of control points and fits a smooth line to connect the control points. Next, a lookup table based on the smooth curve **1102** is generated, i.e., calculated using the fitted function represented by curve **1102,** to facilitate fast mapping from the initial pixel values **1108** of the input image, to the output pixel values 1110 of a normalized image.

Each control point position in the mapping LUT or normalization LUT is a function of the reference intensities of the input image and a pre-determined or assigned output intensity for each reference tissue that closely follows a pre-established relative echogenicity relationship. One such relative echogenicity relationship is that proposed by A.T. Stavros, "Breast Ultrasound", Lippincott Williams and Wilkins, 2004. For the purpose of illustrating the method, the following describes the assignment of a set of control points:

1. Minimum input grey-pixel value is mapped to 0.

2. The intensity of anechoic areas is mapped to the output intensity, $P_{Cyst}*(2^{NrBits} - 1)$, where $P_{Cyst}$ is a predefined percentage value (typically 5%) of the maximum output intensity.

3. The estimated intensity of subcutaneous fat is mapped to the middle intensity of the output dynamic range $(2^{NrBits} - 1)/2$, e.g., 127 for *NrBits=8.*

4. The intensity of skin is recognized to be at the bottom of the range of hyperechoic pixel intensities in the input image, and is mapped to a new intensity, $P_{Skin}*(2^{NrBits} - 1)$, where $P_{Skin}$ is a high, predefined percentage value, such as 90%.

5. The intensity of fibroglandular tissue echogenicities is identified in the midrange of hyperechoic pixel intensities in the input image, and is assigned to the output intensity of $P_{Fibroglandular}*(2^{NrBits} - 1)$, where $P_{Fibroglandular}$ is a predefined percentage value larger than $P_{Skin}$, for example, 95%.

6. The intensity of calcium is estimated to be the average grey pixel value in the very highest intensity clusters of the input image. These input intensities are mapped to $P_{Calcium}*(2^{NrBits} - 1)$ where $P_{Calcium}$ is a predefined percentage value even larger than $P_{Fibrogladular}$, for example, 98%.

7. Finally, the maximum input grey-pixel value is mapped to $(2^{NrBits} - 1)$, e.g., 255 for *NrBits=8.*

The term *NrBits* represents the number of bits used to represent an input pixel intensity value, and a typical value of 8 is presented in the example, so that the dynamic range of an input pixel intensity is from 0 to 255. Larger data types (where the value of *NrBits* may be 16, 32 or 64) or floating point types that support non-integer intensity values could also be used for these purposes.

Figure **11** illustrates the grey-level assignment of pixel intensity values to the respective control point tissues as described above. Although the example here describes a set of seven control points, it will be understood that other suitable sets of control points may be selected and defmed. The selection of control points often depends on imaging modality (e.g., MRI images may require a different set of control points) and anatomic regions being imaged (e.g., images of lungs, prostate or ovaries may be better normalized using a different set of control points).

The normalization LUT can be utilized to normalize the de-noised image at step **306.** The result is a general intensity normalized image. Further steps to process the intensity normalized image are essentially the same as those described earlier in connection with processing a fat normalized image, namely steps **314** and **318** through **324.** The description of these further steps will not be repeated here.

Another variation relates to the use of a malignancy map that may be used to compensate for variations in hardware and operator parameters. As noted earlier, various acquisition parameters involved in an imaging process may affect consistencies of image data. These parameters can be classified into two groups. The first class includes parameters that are due to variations between the ultrasound transducer equipment of different vendors and include depth and transducer frequency. The second class includes factors related to the technologist manual settings such as transducer pressure and TGC.

A malignancy probability map is generated at step **316.** This step may be a replacement of or in addition to generation of a density map (step **314).** The malignancy probability map assigns to each pixel in the input image a probability value of a pixel being malignant. The probability value spans between 0.0 for benign and 1.0 for malignant.

As is known, a probability may have any value between 0 and 1. On the other hand, expert markings indicate lesion areas in a binary fashion: pixels inside a lesion area have a value of 1 while the malignancy value of image background is set to 0. A logistic regression is used to generate a model to deal with binary variables 0 and 1. The model is trained on a large number of images that include lesion areas marked by radiologists. The logistic model is generated incorporating image pixel values and hardware and operator parameters, such as the following:

1. normalized grey pixel value

2. clustered density map value

3. pixel size in mm to indicate the depth of the region of examination from the skin surface

4. transducer frequency

5. TGC value

The malignancy probability map is thus generated taking into account normalized grey pixel values, density map values, and acquisition parameters, thus minimizing the inconsistencies in these parameters.

One major benefit of the logistic model is that it takes into account physical region depth from skin surface, so the resulting malignancy probability map is able to show the top part of a lesion that has a shadowing or a partial shadowing as posterior feature. In contrast, a density map, generally having no information about depth, will not be able to show the posterior feature. Therefore, certain lesion candidates that would be missed by examining the density map alone may be identified from the malignancy probability map.

The malignancy probability map can be clustered at step **318** by applying a predefined probability threshold value to the probability map. All regions that have a probability value larger than the predetermined threshold, such as 0.75, may be grouped into blobs, or "malignancy blobs". Further steps to analyze the malignancy blobs detected and their reporting are similar to those described in connection with the analysis and reporting of density blobs (steps **320** and **322),** and therefore their description will not be repeated here.

Generation of malignancy probability maps is not limited to the logistic model approach described above. The following describes in detail another method of generating a malignancy probability map. This method analyzes features specific to a pixel and features relating to a neighborhood of the pixel to classify a pixel into different tissue types. A probability value that a pixel may belong to each of a set of tissue types is assigned to the pixel, including the probability that a pixel belongs to a lesion, from which a malignancy probability map is generated. This process is described in detail in later sections.

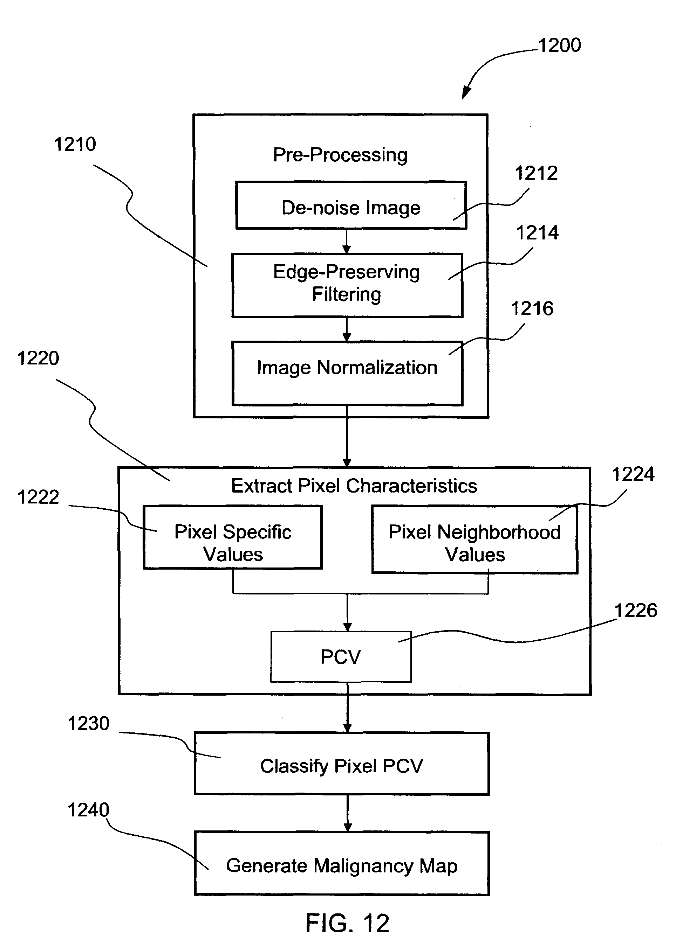

Referring to Figure **12,** an input image is first pre-processed (step **1210).** This may include noise reduction (substep **1212),** edge-preserving filtering (substep **1214)** and image normalization (substep **1216),** not necessarily in this order. The intensities of the input image are normalized (substep **1216)** using dynamically detected control point tissue intensities (such as subcutaneous fat). Anisotropic edge-preserving filtering (substep **1214)** is applied to remove the typical speckle noise from ultrasound images. Filter parameters may be tuned to accommodate vendor specific image characteristics, accounting for the lower native resolution of certain scanners, or increased "inherent smoothing" applied in other scanners. Other pre-processing steps also can be included. For example, the filter tuning may also include factors such as transducer frequency, which often affects the filter configuration for pre-processing.

Next, the method involves computing or constructing a vector, i.e., a set of parameters describing a pixel and its neighborhood (step **1220).** The set of parameters is referred to as a pixel characteristic vector (PCV). Of course, in the case of a 3D image, the set of parameters will describe a voxel and be referred to as a voxel characteristic vector (VCV). The method described herein is equally applicable whether it is a 2D or a 3D image. In the following, no distinction will be made between a PCV and a VCV. Both will be referenced as PCV. Examples of pixel specific feature values include normalized gray level of the pixel and physical depth of the pixel from the skin line. Where available, the approximate position of the pixel may also be included, such as perpendicular distance of the pixel from nipple and angle of the pixel's position from an SI (superior-inferior) line crossing the nipple (the breast clock position). These pixel specific features are extracted from image data at substep **1222** and then included in the pixel's PCV.

Properties of the neighborhood of each pixel are also measured (substep **1224)** in a multi-resolution pixel neighborhood analysis. Several neighborhood sizes and scales are used for analysis, and analysis specifically accounts for the physical size of each pixel (e.g., mm/pixel) at each scale. Several multi-resolution filters are applied to a pixel's neighborhood. The response to the multi-resolution filters provide neighborhood properties of a target pixel, such as texture, edge structure, line-like patterns or grey-level intensities in the neighborhood. Responses of these filters are grouped into categories and included in a neighborhood portion of a PCV. The following examples illustrate filter types that can be applied to assist the identification of tissue types:

1. Line-like pattern filters achieve a high response to linear structures in the image. Pectoralis is often identified by its characteristic short linear "corpuscles", which give it a distinctive texture for which an appropriate sticks filter produces a strong response.

2. Edge filters that generate a strong response at multiple scales are often indicative of long structures. Long hyperechoic lines in the top half of an image may correspond to mammary fascia or cooper ligaments, unless they are horizontal, at the top of the image, in which case they likely indicate skin.

3. Circular hypoechoic regions that are less than 4mm in diameter are generally indicative of healthy ducts or terminal ductile lobular units (TDLUs), so that template circular convolution kernels tend to generate a strong response to these areas.

4. Fourier analysis on large neighborhood sizes indicates the amount of sharp edged detail versus slowly changing shading in a region, and this can be used to identify regions of isoechoic tissue with low frequency texture variations, likely to be fat.

Pixel characteristic vector (PCV) is then constructed for each pixel (substep 1226) from pixel specific values and pixel neighborhood values found at substeps **1222** and **1224.**

After a PCV is found for each pixel, the next step **(1230)** is to classify the PCV of each pixel. Any suitable classifier may be used to classify a PCV. In general, a multi-dimensional classifier component takes the PCV as input, and generates an output vector, which describes the probability with which the pixel in question belongs to each of the specified output classes. The set of output classes may include one or more of the following:

- Fat tissue

- Ducts

- TDLU

- Fascia

- Cooper ligaments

- Lesion tissue

- Pectoralis (muscle) tissue

- Lymph nodes

- Ribs

In one embodiment, a CART tree for classifying PCVs is generated using expert marked training data sets to configure, i.e., to calibrate, the multi-dimensional classifier. A multi-resolution filter set, similar to or the same as the filter set used to find the PCVs, may be applied against those BUS images, in order to tune the filters to generate the maximum discriminating response for each output type. Processing a PCV in the CART tree returns the probability with which the PCV falls into any one of the possible output categories, in particular, the lesion tissue category. After each pixel, i.e., each PCV is classified (step 1230), there is generated a BAM to describe to what type of tissue and anatomy each pixel in a BUS image belongs.

These two steps **1220, 1230** and their substeps, i.e., applying a set of multi-resolution filters to a BUS image to extract the set of PCVs for each pixels and subsequently applying a multi-dimensional classifier to classify the PCVs, may be implemented in the BAM unit **218'**. The BAM unit **218'** may then pass the classified PCVs to the malignancy map unit **218** to extract, or generate, a malignance probability map.

A lesion tissue category indicates that a pixel may be suspicious and belong to an area of the BUS image that warrants further investigation. When classifying a PCV, a probability of the corresponding pixel belonging to lesion tissue is obtained and assigned to the pixel. Malignance probability map is generated (step **1240),** by mapping the malignancy probability values to pixels.

Alternative classification systems may also be used, including neural networks and mixture model (cluster-based) techniques. While the internal process within these other classifiers might be different, all the approaches could be considered as taking the same type of inputs, classifying the pixels, and generating the same type of outputs.

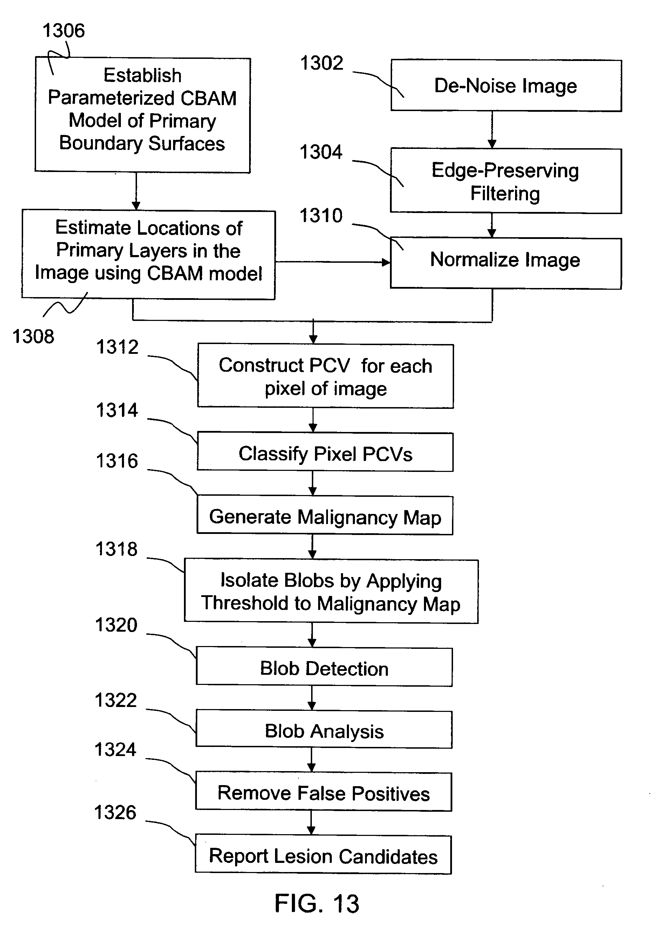

In addition to variations introduced by alternatives to each step of the process shown in Figure **3,** variations to the process shown in Figure **3** are also possible by combining different alternatives described herein. Figure **13** illustrates one such example as an alternative to that shown in Figure **3.**

According to this variation, an image, along with its acquisition parameters, is first retrieved and de-noised (step **1302),** as described before. An edge-preserving filtering is next applied to the de-noised image (step **1304).** Meanwhile, a CBAM model parameterized over $MZD_{max}$ is generated (step **1306).** Detailed steps of building a CBAM model are already described with reference to Figures **6** to **9** and will not be repeated here. Based on an estimated $MZD_{max}$ value of the image, a new CBAM model of the image is generated, from which location of primary layer boundary surfaces can be estimated (step **1308).** The image is next normalized (step **1310),** either using representative intensities of fat alone, or representative intensities of several control point tissues.

As noted earlier, the knowledge of locations of primary layers provided by the output of the CBAM model method can be utilized to improve accuracy and efficiency of subsequent steps. A filter sensitive to a particular type of tissue or lesion may be selectively applied only in a primary layer where the type of tissue or lesion is most likely to occur and not applied in layer or layers where they are not expected. For example, typically, pectoralis muscle has a characteristic texture that is plainly visible in neighborhood larger that 1 mm$^2$ but are typically expected only in the retro-mammary area. For improved efficiency and to reduce false positives, a filter designed to detect pectoralis muscle can be applied only to pixels identified to be in the retro-mammary area. The filter response is set to zero for all other primary layers. This will eliminate the possibility of falsely reporting petoralis in the skin layer. Similarly, filters designed to detect lesions most likely to occur in the mammary zone can be applied to only pixels in the mammary zone, not other layers.

At the next step, when constructing (i.e., computing) a PCV for each of the pixels in the image (step **1312),** the computation can be limited to pixels only in the mammary zone **606** for improved efficiency and accuracy as discussed above. The pixels in the mammary zone are next classified into different tissue types, with a probability value of belonging to each type computed (step **1314).** From probability values of a pixel being malignant, a malignancy map is generated (step **1316).** Next step is to isolate the blobs (step **1318),** for example, by applying a threshold value of the malignancy probability to the malignancy map. Further analysis steps; i.e., blob detection (step **1320),** blob analysis (step **1322)** and removal of false positives (step **1324),** can be carried out. Finally, lesion candidates are reported (step **1326).** These steps all have been described in detail and their descriptions are not repeated here.

**Claims**

1. A method of processing an ultrasound breast image, the method comprising the steps of:

constructing a layered model of breast, each pair of neighboring layers of the model defining a boundary surface between each pair of neighboring layers,

calibrating the model on a plurality of sample ultrasound breast images (720, 730), each of the plurality of sample ultrasound breast images being manually segmented to identify the boundary surfaces in the sample ultrasound breast images, the calibrated model comprising parameterized surface models, each parameterized surface model comprising a set of boundary surface look-up tables i.e. LUTs, corresponding to a discrete value of a size parameter,

receiving an estimated value of the size parameter of the ultrasound breast image (740),

computing a new surface model (750) corresponding to the estimated value of the size parameter from the parameterized surface models, the new surface model comprising a set of computed boundary surface LUTs corresponding to the estimated value of the size parameter, and

computing estimated locations of boundary surfaces (760) from the set of computed boundary surface LUTs of the new surface model to identify pixels of a primary layer in the ultrasound breast image.

2. The method of claim 1, further comprising:

finding representative intensity values of a plurality of control point tissues, the representative intensity values of the plurality of control point tissues including a representative intensive value of grey pixels values of subcutaneous fat layer, and

deriving a normalized image by normalizing the ultrasound breast image with respect to the plurality of control point tissues based on a mapping relationship between the representative intensity values of the plurality of control point tissues and normalized values of the plurality of control point tissues.

3. The method of claims 1 or claim 2, wherein the layered model includes skin layer and retro-mammary layer, the method further comprising:

estimating locations of a first boundary surface demarcating the skin layer and a second boundary surface demarcating the retro-mammary layer, and

rendering a portion of the ultrasound breast image for visualization, the portion of the ultrasound breast image excluding the skin layer and the retro-mammary layer.

4. The method of any preceding claim, wherein the primary layer is a mammary zone, and the method further comprising:

for each pixel in the mammary zone, estimating a probability value of the each pixel being malignant,

grouping pixels with probability values above a threshold value into contiguous regions as suspect lesions.

5. A system for processing an ultrasound breast image comprising:

means for constructing a layered model of breast, each pair of neighboring layers of the model defining a boundary surface between each pair of neighboring layers,

means for calibrating the model on a plurality of sample ultrasound breast images (720, 730), each of the plurality of sample ultrasound breast images being manually segmented to identify the boundary surfaces in the sample ultrasound breast images, the calibrated model comprising parameterized surface models, each parameterized surface model comprising a set of boundary surface look-up tables i.e. LUTs, corresponding to a discrete value of a size parameter,

means for receiving an estimated value of the size parameter of the ultrasound breast image (740),

means for computing a new surface model (750) corresponding to the estimated value of the size parameter from the parameterized surface models, the new surface model comprising a set of computed boundary surface LUTs corresponding to the estimated value of the size parameter, and

means for computing estimated locations of boundary surfaces (760) from the set of computed boundary surface LUTs of the new surface model to identify pixels of a primary layer in the ultrasound breast image.

**Patentansprüche**

1.  Verfahren zum Verarbeiten eines Ultraschallbrustbildes, wobei das Verfahren die Schritte aufweist:

    Konstruieren eines geschichteten Modells einer Brust, wobei jedes Paar benachbarter Schichten des Modells eine Grenzfläche zwischen jedem Paar benachbarter Schichten definiert,
    Kalibrieren des Modells an mehreren Ultraschall-Brustprobebildern (720, 730), wobei jedes der mehreren Ultraschall-Brustprobebilder manuell segmentiert wird, um die Grenzflächen in den Ultraschall-Brustprobebildern zu identifizieren, wobei das kalibrierte Modell parametrisierte Oberflächenmodelle aufweist, wobei jedes parametrisierte Oberflächenmodell einen Satz von Grenzflächen-Verweistabellen, d.h. LUTs (look-up tables), aufweist, die einem diskreten Wert eines Größenparameters entsprechen,
    Empfangen eines Schätzwertes des Größenparameters des Ultraschallbrustbildes (740),
    Berechnen eines neuen Oberflächenmodells (750) entsprechend dem Schätzwert des Größenparameters aus den parametrisierten Oberflächenmodellen, wobei das neue Oberflächenmodell einen Satz berechneter Grenzflächen-LUTs entsprechend dem Schätzwert des Größenparameters aufweist, und
    Berechnen geschätzter Positionen von Grenzflächen (760) aus dem Satz berechneter Grenzflächen-LUTs des neuen Oberflächenmodells zur Identifizierung von Pixeln einer primären Schicht in dem Ultraschallbrustbild.

2.  Verfahren nach Anspruch 1, des Weiteren aufweisend:

    Ermitteln repräsentativer Intensitätswerte mehrerer Kontrollpunktgewebe, wobei die repräsentativen Intensitätswerte der mehreren Kontrollpunktgewebe einen repräsentativen Intensitätswert von Graupixelwerten einer subkutanen Fettschicht enthalten, und Ableiten eines normalisierten Bildes durch Normalisieren des Ultraschallbrustbildes in Bezug auf die mehreren Kontrollpunktgewebe auf der Basis eines Abbildungsverhältnisses zwischen den repräsentativen Intensitätswerten der mehreren Kontrollpunktgewebe und normalisierten Werten der mehreren Kontrollpunktgewebe.

3.  Verfahren nach Anspruch 1 oder Anspruch 2, wobei das geschichtete Modell eine Hautschicht und eine retro-mamillarische Schicht enthält, wobei das Verfahren des Weiteren aufweist:

    Schätzen von Positionen einer ersten Grenzfläche, die die Hautschicht abgrenzt, und einer zweiten Grenzfläche, die die retro-mamillarische Schicht abgrenzt, und
    Wiedergeben eines Teils des Ultraschallbrustbildes zur Visualisierung, wobei der Teil des Ultraschallbrustbildes die Hautschicht und die retro-mamillarische Schicht ausschließt.

4.  Verfahren nach einem vorangehenden Ansprüche, wobei die primäre Schicht eine Mammazone ist und wobei das Verfahren des Weiteren aufweist:

    für jedes Pixel in der Mammazone Schätzen eines Wahrscheinlichkeitswertes, dass jedes Pixel maligne ist,
    Gruppieren von Pixeln mit Wahrscheinlichkeitswerten über einem Schwellenwert in angrenzende Regionen als verdächtige Läsionen.

5.  System zum Verarbeiten eines Ultraschallbrustbildes, aufweisend:

    Mittel zum Konstruieren eines geschichteten Modells einer Brust, wobei jedes Paar benachbarter Schichten des Modells eine Grenzfläche zwischen jedem Paar benachbarter Schichten definiert,
    Mittel zum Kalibrieren des Modells an mehreren Ultraschall-Brustprobebildern (720, 730), wobei jedes der mehreren Ultraschall-Brustprobebilder manuell segmentiert wird, um die Grenzflächen in den Ultraschall-Brustprobebildern zu identifizieren, wobei das kalibrierte Modell parametrisierte Oberflächenmodelle aufweist, wobei jedes parametrisierte Oberflächenmodell einen Satz von Grenzflächen-Verweistabellen, d.h. LUTs (look-up tables), aufweist, die einem diskreten Wert eines Größenparameters entsprechen,
    Mittel zum Empfangen eines Schätzwertes des Größenparameters des Ultraschallbrustbildes (740),
    Mittel zum Berechnen eines neuen Oberflächenmodells (750) entsprechend dem Schätzwert des Größenparameters aus den parametrisierten Oberflächenmodellen, wobei das neue Oberflächenmodell einen Satz berechneter Grenzflächen-LUTs entsprechend dem Schätzwert des Größenparameters aufweist, und
    Mittel zum Berechnen geschätzter Positionen von Grenzflächen (760) aus dem Satz berechneter Grenzflächen-LUTs des neuen Oberflächenmodells zur Identifizierung von Pixeln einer primären Schicht in dem Ultraschallbrustbild.

**Revendications**

1. Procédé de traitement d'une image ultrason mammaire, le procédé comprenant les étapes ci-dessous consistant à :

construire un modèle de poitrine agencé en couches, chaque paire de couches voisines du modèle définissant une surface limite entre chaque paire de couches voisines ;

étalonner le modèle d'après une pluralité d'images ultrasons mammaires d'échantillonnage (720, 730), chaque image de la pluralité d'images ultrasons mammaires d'échantillonnage étant segmentée manuellement pour identifier les surfaces limites dans les images ultrasons mammaires d'échantillonnage, le modèle étalonné comprenant des modèles de surface paramétrés, chaque modèle de surface paramétré comprenant un ensemble de tables de consultation de surfaces limites, autrement dit des tables LUT, correspondant à une valeur discrète d'un paramètre de taille ;

recevoir une valeur estimée du paramètre de taille de l'image ultrason mammaire (740) ;

calculer un nouveau modèle de surface (750) correspondant à la valeur estimée du paramètre de taille à partir des modèles de surface paramétrés, le nouveau modèle de surface comprenant un ensemble de tables LUT de surfaces limites calculées correspondant à la valeur estimée du paramètre de taille ; et

calculer des emplacements estimés de surfaces limites (760) à partir de l'ensemble de tables LUT de surfaces limites calculées du nouveau modèle de surface pour identifier des pixels d'une couche primaire dans l'image ultrason mammaire.

2. Procédé selon la revendication 1, consistant en outre à :

trouver des valeurs d'intensité représentatives d'une pluralité de tissus de point de contrôle, les valeurs d'intensité représentatives de la pluralité de tissus de point de contrôle incluant une valeur intensive représentative de valeurs de pixels gris de couche de graisse sous-cutanée ; et

dériver une image normalisée en normalisant l'image ultrason mammaire relativement à la pluralité de tissus de point de contrôle, sur la base d'une relation de mise en correspondance entre les valeurs d'intensité représentatives de la pluralité de tissus de point de contrôle et des valeurs normalisées de la pluralité de tissus de point de contrôle.

3. Procédé selon la revendication 1 ou 2, dans lequel le modèle en couches inclut une couche cutanée et une couche rétromammaire, le procédé consistant en outre à :

estimer des emplacements d'une première surface limite délimitant la couche cutanée, et d'une seconde surface limite délimitant la couche rétromammaire ; et

mettre en oeuvre un rendu d'une partie de l'image ultrason mammaire à des fins de visualisation, la partie de l'image ultrason mammaire excluant la couche cutanée et la couche rétromammaire.

4. Procédé selon l'une quelconque des revendications précédentes, dans lequel la couche primaire est une zone mammaire, et le procédé consiste en outre à :

pour chaque pixel dans la zone mammaire, estimer une valeur de probabilité que chaque dit pixel soit malin ;

regrouper des pixels dont les valeurs de probabilité sont supérieures à une valeur de seuil dans des zones contiguës, en tant que lésions suspectes.

5. Système destiné à traiter une image ultrason mammaire, comprenant :

des moyens pour construire un modèle de poitrine agencé en couches, chaque paire de couches voisines du modèle définissant une surface limite entre chaque paire de couches voisines ;

des moyens pour étalonner le modèle d'après une pluralité d'images ultrasons mammaires d'échantillonnage (720, 730), chaque image de la pluralité d'images ultrasons mammaires d'échantillonnage étant segmentée manuellement pour identifier les surfaces limites dans les images ultrasons mammaires d'échantillonnage, le modèle étalonné comprenant des modèles de surface paramétrés, chaque modèle de surface paramétré comprenant un ensemble de tables de consultation de surfaces limites, autrement dit des tables LUT, correspondant à une valeur discrète d'un paramètre de taille ;

des moyens pour recevoir une valeur estimée du paramètre de taille de l'image ultrason mammaire (740) ;

des moyens pour calculer un nouveau modèle de surface (750) correspondant à la valeur estimée du paramètre de taille à partir des modèles de surface paramétrés, le nouveau modèle de surface comprenant un ensemble de tables LUT de surfaces limites calculées correspondant à la valeur estimée du paramètre de taille ; et
des moyens pour calculer des emplacements estimés de surfaces limites (760) à partir de l'ensemble de tables LUT de surfaces limites calculées du nouveau modèle de surface, pour identifier des pixels d'une couche primaire dans l'image ultrason mammaire.

100

110
Receive Image Data

120
De-noise Image

130
Image Normalization

140
Blob Detection

150
Feature Based Blob Analysis

160
Remove False Positives

170
Lesion Report

FIG. 1

FIG. 2

300

302

Receive Image and
Acquisition Data

308

Fat Intensity
Measurement

304

Noise Reduction and
Removal

310

Control Points
Intensities

306

Intensity
Normalization

312

Find Mapping
LUT

316

Malignancy Map

314

Density Map

318

Blob Detection

320

Feature Based Blob
Analysis

322

Remove False Positives

324

Lesion Report

FIG. 3

FIG. 4A

FIG. 4B

500

502

**Input Ultrasound Image**

504

**Identify Fat Region**

506

**Apply k-means Clustering to Fat Region, with k=3**

508

**Extract Mid-Level Cluster Intensities**

510

**Compute Fat Intensity**

512

**Normalize Image**

FIG. 5

FIG. 6

700

710

**First Stage**

Receive Segmented Sample Images and Scanning Acquisition Parameters

720

Calculate Locations of Boundary Surfaces in Each Sample Image

730

Compute a Set of Surface LUTs for Each Discrete $MZD_{max}$ in a Range

**Second Stage**

740

Determine an estimated $MZD_{max}$ value of the image

750

Compute a set of Surface LUTs for the estimated $MZD_{max}$ value

**Third Stage**

760

Estimate Locations of Boundary Surfaces in the image

**FIG. 7**

FIG. 8

FIG. 9A

FIG. 9B

FIG. 10A

FIG. 10B

FIG. 11

1200

1210

Pre-Processing

1212

De-noise Image

1214

Edge-Preserving
Filtering

1216

Image Normalization

1220

Extract Pixel Characteristics

1224

1222

Pixel Specific
Values

Pixel Neighborhood
Values

1226

PCV

1230

Classify Pixel PCV

1240

Generate Malignancy Map

FIG. 12

1306

Establish
Parameterized CBAM
Model of Primary
Boundary Surfaces

1302 → De-Noise Image

1304 → Edge-Preserving
Filtering

Estimate Locations of
Primary Layers in the
Image using CBAM model

1310 → Normalize Image

1308

1312 → Construct PCV for each
pixel of image

1314 → Classify Pixel PCVs

1316 → Generate Malignancy Map

1318 → Isolate Blobs by Applying
Threshold to Malignancy Map

1320 → Blob Detection

1322 → Blob Analysis

1324 → Remove False Positives

1326 → Report Lesion Candidates

FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2006057740 A

### Non-patent literature cited in the description

- **R.O. DUDA.** Pattern Classification. John Wiley & Sons Inc, 2001
- Anisotropic Diffusion in Image Processing. **WEICKERT, J.** ECMI Series. Verlag, 1998
- **A.T. STAVROS.** Breast Ultrasound. Lippincott Williams and Wilkins, 2004